# EUROPEAN PATENT APPLICATION

(11) **EP 4 085 901 A1**
(43) Date of publication of application: **09.11.2022**
(21) Application number: 19958197.6
(22) Date of filing: 31.12.2019
(51) Int. Cl.: A61K 9/16, A61K 31/519, A61K 31/496, A61P 25/18, A61K 47/34

(54) **TERTIARY AMINE PHARMACEUTICAL COMPOSITION AND INDUSTRIAL BATCH PREPARATION METHOD THEREFOR**

(71) Applicant: Guangzhou DiQi Pharmaceuticals Co., Ltd., Guangzhou International BIO-island Guangzhou Guangdong 510300 (CN)
(72) Inventor: LAI, Shuting, Guangzhou, Guangdong 510300 (CN); ZHENG, Yang, Guangzhou, Guangdong 510300 (CN); CAO, Fuchun, Guangzhou, Guangdong 510300 (CN); LIAN, Yuanfa, Guangzhou, Guangdong 510300 (CN); LIU, Feng, Guangzhou, Guangdong 510300 (CN)
(74) Representative: Murgitroyd & Company
(86) International application number: PCT/CN2019/130833
(87) International publication number: WO 2021/134623

(57) **Abstract**

Provided is a tertiary amine pharmaceutical composition, comprising a drug having a tertiary amine structure, a biocompatible polymer material, and a quaternary ammonium salt impurity. The pharmaceutical composition is obtained by dissolving or dispersing the drug in a halogenated hydrocarbon or a mixed solvent mainly containing halogenated hydrocarbon or a solution containing halogenated hydrocarbon, and the quaternary ammonium salt impurity is generated from reacting the drug having the tertiary amine structure with the halogenated hydrocarbon, wherein the pharmaceutical composition comprises 40wt% to 80wt% of the biocompatible polymer material, and 20wt % to 60wt% of the drug with the tertiary amine structure; the content of the quaternary ammonium salt impurity is less than 0.05wt%; and the content of the halogenated hydrocarbon in the composition is less than 1.5wt%, and the quaternary ammonium salt impurity does not increase or increase slowly during storage, complying with requirements of pharmaceutical regulations.

## Description

### TECHNICAL FIELD

The present invention belongs to the field of pharmaceuticals, in particular to a tertiary amine pharmaceutical composition and an industrialized batch preparation method.

### BACKGROUND

At present, most preparation methods of sustained and controlled release compositions need organic solvents for dissolving or dispersing. For example, both a microsphere preparation method disclosed in patent EP0052510A and a microsphere preparation method disclosed in patent US3976071A are based on the premise of dissolving water-insoluble polymers with organic solvents. For sustained-release compositions prepared by such methods, there is a consensus that residue of the organic solvents will lead to toxicity and affect the release behavior of the compositions. However, there are few reports on the influence of impurities introduced by the organic solvents and the increase of impurities during storage.

Menshutkin reaction is a reaction between tertiary amine and a halogenated hydrocarbon to generate a quaternary ammonium salt. As most biodegradable polymer materials (such as PLGA) used in sustained-release preparations have high solubility in halogenated hydrocarbons, the halogenated hydrocarbons, especially dichloromethane and trichloromethane, are the most common solvent in the current preparation methods of sustained-release preparations (such as emulsion-solvent evaporation method). For drug with a tertiary amine structure, it is very likely that a Menshutkin reaction occurs between the drug and the halogenated hydrocarbon to generate a quaternary ammonium salt impurity in this system. The impurity produced in the preparation process belongs to a process impurity. According to ICH guiding principle, non-specific impurities in new drug preparations should be controlled below 0.2wt%. Meanwhile, the quaternary ammonium salt is generally used as a disinfectant and a fungicide, and different structures have strong or weak toxic and side effects on human bodies, which may also have great influence on the efficacy, pharmacodynamics and toxicology of the original drug. Therefore, it is necessary to control the impurities generated by the reaction between tertiary amine drugs and halogenated hydrocarbons.

Meanwhile, it is also found in the present application that if the quaternary ammonium salt impurities produced by the Menshutkin reaction between the drug with tertiary amine structure and the halogenated hydrocarbon exceeds a certain amount, the quaternary ammonium salt impurities will continue to increase during storage, especially if the residual amount of the halogenated hydrocarbons exceeds a certain amount, the increase is more significant, which is not conducive to the long-term storage of the pharmaceutical composition, and may cause adverse reactions after administration.

### SUMMARY

Object of the present invention: aiming at the problems in the prior art, the present invention provides a tertiary amine pharmaceutical, which can avoid a problem of continuously increased quaternary ammonium salt impurity during storage composition by controlling a content of the quaternary ammonium salt impurity and an amount of residue of a halogenated hydrocarbon in the tertiary amine pharmaceutical composition, and meanwhile, the drug storage conditions are also more inclusive.

In order to solve the foregoing technical problems, the present invention employs the following technical solutions.

A tertiary amine pharmaceutical composition comprises a drug with a tertiary amine structure, a biocompatible polymer material, and a quaternary ammonium salt impurity. The pharmaceutical composition is obtained by dissolving or dispersing the drug in a halogenated hydrocarbon or a mixed solvents mainly containing halogenated hydrocarbon or a solution containing halogenated hydrocarbon, and the quaternary ammonium salt impurity is generated from reaction of the drug having tertiary amine structure with the halogenated hydrocarbon, wherein the pharmaceutical composition comprises 40wt% to 80wt% of the biocompatible polymer material, and 20wt % to 60wt% of the drug having the tertiary amine structure; a content of the quaternary ammonium salt impurity is less than 0.05wt%; and a content of the halogenated hydrocarbon in the tertiary amine pharmaceutical composition is less than 1.5wt%, in some embodiments, the content of the halogenated hydrocarbon in the tertiary amine pharmaceutical composition is less than 1.25wt%.

The drug with tertiary amine structure is directly contacted with the halogenated hydrocarbon in the preparation process steps. Further, the drug with tertiary amine structure is completely dissolved, partially dissolved or slightly dissolved in the halogenated hydrocarbon. The completely dissolved means that a mass concentration x is greater than 1%, the partially dissolved means that the mass concentration is that 0.1%≤x≤1%, and the slightly dissolved means that x is less than 0.1%.

The drug with tertiary amine structure comprises any one of entecavir, oxybutynin, raloxifene, rivastigmine, naloxone, naltrexone, buprenorphine, selegiline, buspirone, blonanserin, asenapine, olanzapine, lurasidone, 3-[2-[4-(6-fluoro-1,2-benzisoxazole-3-yl)-1-piperidinyl] ethyl]-6,7,8,9-tetrahydro-2-methyl-4H-pyridine[1,2-α]pyrimidine-4-keton, 6,7,8,9-tetrahydro-3-(2-(4-(6-fluoro-1,2-benzisoxazole-3-yl)-1-piperidinyl)ethyl)-9-hydroxy-2-methyl-4H-pyridine[2,1-a]pyrimidine-4-one and (±)-3-[2-[4-[6-fluoro-1,2-benzisoxazole-3-yl)-1-piperidine]ethyl]-6,7,8,9-tetrahydro-2-methyl-4-oxo-4H-py ridine[1,2-a]pyrimidine-9-yl palmitate.

In view of the reaction generation principle of the quaternary ammonium salt impurities, in order to control the content of the quaternary ammonium salt impurities in a certain range and keep the quaternary ammonium salt impurities not increase or increase slowly at conventional storage temperature, the technical solutions of the present invention are also applicable to other drugs with tertiary amine structure, for example:
the technical solutions are also applicable to phentolamine, amiodarone, azithromycin, chlorambucil, diltiazem, dimenhydrinate, diphenhydramine, erythromycin, chlormethine, melphalan, methadone, propoxyphene, tamoxifen, terbinafine, tetracycline, clonidine, clomiphene, donepezil, cabergoline, selegiline, buspirone, loxapine, amisulpride, aripiprazole, dehydroaripiprazole, bifeprunox, clopenthixol, haloperidol, tandospirone, fluphenazine, quetiapine, remoxipride, zotepine, pimozide, promethazine, tetrabenazine, hydroxytetrabenazine, cariprazine, citalopram, galantamine, iloperidone, clozapine, escitalopram, perospirone, ziprasidone, amitriptyline, doxepin, triflupromazine, trimipramine, clomipramine, imipramine, mianserin, mirtazapine, ritanserin, trazodone, venlafaxine, and the like.

The biocompatible polymer material is PLGA, and other biocompatible polymer materials such as PCL, R-PEG-PLGA, PLGA-PEG-PLGA, PCL-PLGA, R-PEG-PLA, PLA-PEG-PLA and PCL-PLA are also applicable, wherein R is H, OH, Me, NH₂, FA or OMe.

Preferably, the biocompatible polymer material is PLGA, a ratio of LA to GA of the PLGA is 100/0 to 50/50, a molecular weight of the PLGA is 20 kDa to 60 kDa, and an intrinsic viscosity of the PLGA is 0.2 dL/g to 0.65 dL/g.

In the tertiary amine pharmaceutical composition, the content of the halogenated hydrocarbon is no more than 1.5wt%, preferably no more than 1.25wt%, and more preferably no more than 1wt%.

In some embodiments, the drug with tertiary amine structure is any one of N1, N2 or N3, the biocompatible polymer material is PLGA, and the halogenated hydrocarbon is a solvent comprising at least one of chloroform or dichloromethane; the content of the quaternary ammonium salt impurities is less than 0.04wt%; and the content of the dichloromethane or the trichloromethane is no more than 1.25wt%, preferably no more than 1wt%, and more preferably no more than 0.7wt%.

In some embodiments, the ratio of LA to GA of the PLGA is 100/0 to 70/30, the molecular weight of the PLGA is 20 kDa to 55 kDa, and the intrinsic viscosity of the PLGA is 0.2 dL/g to 0.57 dL/g.

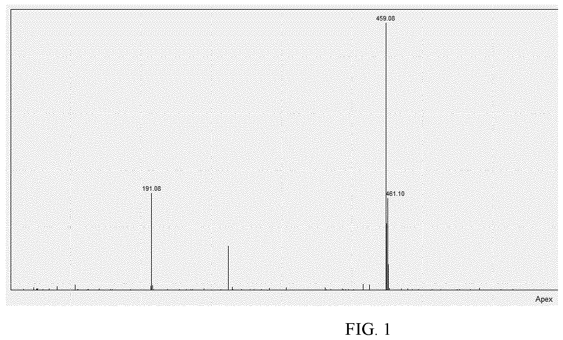

In other embodiments, the drug with tertiary amine structure is N1 with a content of 30wt% to 50wt%; the biocompatible polymer material is PLGA with a content of 50wt% to 70wt%; and a structural formula of the impurity is as shown by Formula (1): 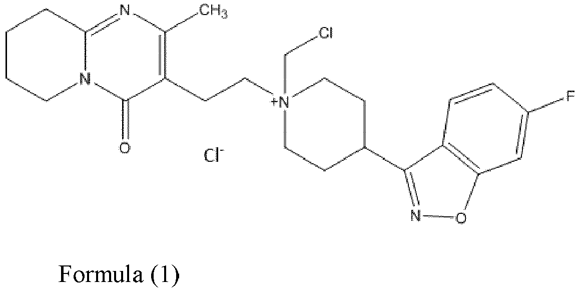 wherein, the content of the impurity is less than 0.03wt%, and the mass spectrogram of the impurity is as shown in FIG. 1.

Preferably, the content of the impurity shown in the mass spectrogram FIG. 1 in the tertiary amine pharmaceutical composition is less than 0.02wt%, and more preferably, less than 0.01wt%.

In some embodiments, the ratio of LA to GA of the PLGA is 95/05 to 70/30, the molecular weight of the PLGA is 25 kDa to 50 kDa, and the intrinsic viscosity of the PLGA is 0.24 dL/g to 0.52 dL/g. Preferably, in the tertiary amine pharmaceutical composition above, the content of the dichloromethane is no more than 1. 1wt%, preferably no more than 0.9wt%, and more preferably no more than 0.7wt%.

The present invention further provides an HPLC detection method for an impurity generated by a reaction between N1 and dichloromethane, which is as follows:
(1) chromatographic column: Agilent ZORBAX SB-C18, 150×4.60 mm, 5 µm or equivalent chromatographic column;
(2) mobile phase: acetonitrile: trifluoroacetic acid: water = 20: 0.15: 80 (pH 3.0)
(3) flow rate: 1.5 mL/min
(4) column temperature: 35 °C
(5) detection wavelength: 275 nm

Under these detection conditions, the RRT of the impurity is 1.5.

Under these detection conditions, there may also be known impurities U1, U2 and U3 in the N1 pharmaceutical composition, with RRT of 0.7, 1.6 and 1.7 respectively. It shows that the generation and change of these impurities are not directly related to the existence and content of the halogenated hydrocarbon.

The tertiary amine pharmaceutical composition above can be a block, a strip or particle; preferably, the tertiary amine pharmaceutical composition is a nano-particle, a submicron particle, a micron particle or a millimicron particle; preferably, the tertiary amine pharmaceutical composition is a spherical or non-spherical particle with a major axis of 10 µm to 200 µm; and more preferably, the tertiary amine pharmaceutical composition is a spherical or non-spherical particle with a major axis of 25 µm to 150 µm.

The present invention further provides an application of the tertiary amine pharmaceutical composition in preparing a drug for resisting anxiety and depression, and treating acute and chronic schizophrenia and other obvious positive and negative symptoms of various psychotic states, wherein the drug with tertiary amine structure is selected from buspirone, loxapine, amisulpride, aripiprazole, dehydroaripiprazole, bifeprunox, blonanserin, clopenthixol, tandospirone, fluphenazine, haloperidol, quetiapine, remoxipride, zotepine, promethazine, tetrabenazine, hydroxytetrabenazine, asenapine, cariprazine, iloperidone, olanzapine, clozapine, escitalopram, lurasidone, perospirone, ziprasidone, amitriptyline, doxepin, imipramine, triflupromazine, trimipramine, clomipramine, mianserin, mirtazapine, ritanserin, trazodone, venlafaxine, N1, N2 or N3, and is usd for resisting anxiety and depression, and treating the acute and chronic schizophrenia and other obvious positive and negative symptoms of various psychotic states.

In some embodiments, the tertiary amine pharmaceutical composition, which is the spherical or non-spherical particle, is dispersed or suspended in a solvent during administration, and then administered subcutaneously or intramuscularly. The solvent comprises the following components: a nonionic surfactant (or stabilizer) (such as polyoxyethylene castor oil derivative, cellulose thickener, sodium alginate, hyaluronic acid, dextrin and starch), an isotonic agent (such as sodium chloride, mannitol, glycerol, sorbitol, lactose, xylitol, maltose, galactose, sucrose, glucose, and the like), a pH regulator (such as carbonic acid, acetic acid, oxalic acid, citric acid, phosphoric acid and hydrochloric acid or salts of these acids, such as sodium carbonate, sodium bicarbonate, and the like), and a preservative (such as paraben, propyl p-hydroxybenzoate, benzyl alcohol, chlorobutanol, sorbic acid, boric acid, and the like). The above components are made into aqueous solution, then solidified by freeze-drying, vacuum drying, spray drying, and the like, and re-dissolved with water for injection before use.

The solvent has a viscosity ranging from 20 mPa.s to 45 mPa.s, a pH value ranging from 6.5 to 7.5, and an osmotic pressure ranging from 270 mOsmol/kg to 290 mOsmol/kg.

The tertiary amine pharmaceutical composition can be continuously and slowly released for no less than two weeks, four weeks, eight weeks, twelve weeks, and the like, in vitro simulating body fluids or in vivo.

The tertiary amine pharmaceutical composition above may be prepared by any one of normal or low temperature spray freeze-drying method, emulsion-solvent evaporation method, phase separation method and supercritical fluid method.

The present invention also provides an industrialized batch preparation method of the tertiary amine pharmaceutical composition. The industrialized batch refers to a production batch that meets the commercial supply demand for a certain period of time, and is related to a single batch production cycle, or a number of patients, or a mode of administration, or a supply chain capacity. The industrialized batch of the tertiary amine pharmaceutical composition with sustained-controlled release effects of the present invention refers to a batch with a single batch production cycle (covering manufacturing, packaging, inspection, quality control and other necessary processes before marketing) of 1 month /2 months, with no less than 500,000 doses, preferably no less than one million doses, and more preferably no less than five million doses.

Specifically, the industrialized batch preparation method uses an emulsion-solvent evaporation method, comprising the following steps of:
(1) preparing an aqueous solution with a surfactant, and cooling the aqueous solution for later use, wherein a temperature of the solution is 5°C±3°C;
(2) dissolving or dispersing the drug with tertiary amine structure and the biocompatible polymer material in halogenated hydrocarbon to form a solution or suspension, wherein a time spent is no more than 4 hours, a temperature of the solution shall not exceed 20°C±5°C, and no heating step is carried out;
(3) injecting the solution or suspension obtained in step (2) into the aqueous solution with the surfactant for emulsification to form O/W or S/O/W emulsion, wherein a time spent is no more than 4 hours, an ambient temperature shall not exceed 25°C, and no heating or vacuum step is carried out;
(4) mechanically stirring to remove the halogenated hydrocarbon in a product of step (3) to obtain a solidified spherical or non-spherical particle, wherein a duration for the mechanical stirring is no less than 24 hours, an ambient temperature and a system temperature shall not exceed 25°C, no heating or vacuum step is carried out, and a residual amount of the halogenated hydrocarbon in the obtained particle is no more than 2.0wt%;
(5) washing the particle of step (4) to remove the surfactant on the surface of the particles; and
(6) freeze-drying the particle of step (5) to obtain the tertiary amine pharmaceutical composition.

Preferably, step (2) and step (3) are continuous processes, or a time between the two steps is no more than 1 hour.

Preferably, in step (5), the particle of step (4) is washed with water for injection at 5 times the volume not exceeding 10°C to remove the polyvinyl alcohol on the surface of the particles.

Specifically, in step (6), freeze-drying conditions are:
pre-freezing at -30°C to -45°C for 4 hours to 6 hours; primary drying at -20°C to -5°C for 8 hours to 16 hours under 100 mtorr to 200 mtorr; secondary drying at 30°C to 35°C for 18 hours to 30 hours under 100 mtorr to 200 mtorr; and the residual amount of the halogenated hydrocarbon in the obtained particle being no more than 1.5wt%

Direct contact is occured when the drug with tertiary amine structure is dissolved and dispersed in dichloromethane, which provides conditions for the reaction. For the production batch, the amount of polymer used reaches kilogram level, and the dissolving process in step (2) takes a certain time because the polymer cannot be heated; a volume of the dissolved solution reaches several hundred liters, which also takes a certain time to filter. In the emulsification process, O and W are emulsified in a high-speed homogenizer in proportion, and the whole emulsification step also needs to last for a certain time. Therefore, it is necessary to control the time and the temperature of steps (2) and (3) to avoid or reduce a reaction between the drug and the halogenated hydrocarbon, thus reducing the generation of reaction impurities.

Only when the content of the dichloromethane is reduced to a certain level can a reaction between the drug and the dichloromethane be reduced or avoided. Step (4) affects the residual amount of the dichloromethane in the composition, which is related to the duration time of this step. Within a certain range, the longer the time is, the lower the content of the dichloromethane is, thus the lower the possibility of reaction with the drug with tertiary amine structure in the composition is, and the less the impurities are.

In the N1 composition, a content of N1 is 36wt%, and a single dosage is 50 mg to 100 mg. When a yield is 80% and a concentration of N1 is 10%, there are 500,000 dose batches, and the volume of the solution in step (2) is about 500 L to 1,000 L. Under the high-speed homogenization step, a flow rate of O phase to a flow rate of W phase is 1:100, and the flow rate of W phase is 50,000 L to 100,000 L. According to literature reports, under the same conditions, the smaller the flow rate is, the better the emulsification effect is and the narrower the particle size distribution is, because if the flow rate is larger, the contact time between the solution and the homogenizer head is shorter, which will lead to the decrease of the homogenization effect and the wider particle size distribution of the emulsion.

According to the high-speed homogenizer (IKNERS2000, Shanghai Yiken Machinery Equipment Co., Ltd., wherein a flow rate of the model above 3,000 rpm is 300 L/h to 20,000 L/h; or IKA ULTRA-TURRAX UTL2000, IKA Works (Guangzhou), wherein a throughput of models above 3,000 rpm is 50 L/h to 40,000 L/h), 2.5 hours to 5 hours are needed to complete the emulsification process at full load. If the condition is optimized as 50% flow rate, it will take 5 hours to 10 hours. The smaller the optimized flow rate is, the longer the total emulsification time is. When the batch is increased to more than one million doses, the whole emulsification time of a single production line needs more than 10 hours. However, the longer the emulsification time is, the longer the contact time between the drug and the halogenated hydrocarbon is, and the more the impurities are produced. Therefore, when the batch size is higher, multiple high-speed homogenizers are needed to emulsify at the same time to shorten the process time, which also leads to higher equipment cost and research contents of process stability.

The surfactant is polyvinyl alcohol.

In a specific embodiment, the present invention provides an industrialized batch preparation method of a tertiary amine pharmaceutical composition, wherein a drug with tertiary amine structure is N1, and the method comprises the following steps of:
(1) preparing 0.1wt% to 1.5wt% polyvinyl alcohol aqueous solution, and cooling to 5°C+/-3°C for later use;
(2) after the step (1) is completed, completely dissolving N1 and PLGA in dichloromethane under mechanical stirring, and then filtering the mixture by a filter membrane with a pore diameter of 0.2 µm;
(3) slowly injecting the solution of step (2) into the solution of step (1) for high-speed homogenization and emulsification to form an O/W emulsion;
(4) mechanically stirring the emulsion of step (3) to remove the dichloromethane, and then filtering to obtain a solidified spherical or non-spherical particles,
(5) washing the particle of step (4) with water for injection at 5 times the volume not exceeding 10°C to remove the polyvinyl alcohol on a surface of the particles; and
(6) freeze-drying the particles of step (5) to obtain the composition.

Wherein:
a duration spent in step (2) is no more than 3 hours, a temperature of the solution is no more than 20°C, and no heating step is carried out;
in step (3), a flow ratio of an O phase to a W phase is 1:100, a total emulsification time shall not exceed 4 hours, an ambient temperature shall not exceed 25°C, and no heating or vacuum step is carried out;
step (2) and step (3) are continuous processes;
a duration of step (4) is no less than 36 hours, an ambient temperature and a system temperature shall not exceed 25°C, no heating or vacuum step is carried out, and a residual amount of the halogenated hydrocarbon in the obtained particle is no more than 1.5wt%; and
the residual amount of the halogenated hydrocarbon in the particle obtained in step is no more than 1.25wt%, and the freeze-drying method is:

| | |
|---|---|
| pre-freezing | -30°C to -45°C, 4 hours to 6 hours |
| primary drying | -20°C to -5°C, 8 hours to 16 hours, 100 mtorr to 200 mtorr; |
| secondary drying | 30°C to 35°C, 18 hours to 30 hours, 100 mtorr to 200 mtorr |

Preferably, in the curing process of step (4), the particles are filtered for equal time and replaced by the same volume of room temperature water for 2 times to 3 times.

A dosage of the N1 composition is 50 mg to 100 mg, and the N1 composition is administered once every four weeks or one month. In some embodiments, the dosage of the N1 composition is 100 mg to 200 mg, and the N1 composition is administered once every eight weeks or two months.

For simple preparations (such as tablets), most of the simple preparations may be prepared at room temperature or under cool conditions. For complex preparations (such as biological preparations and long-acting injections), most of the complex preparations need to be stored under harsh conditions, such as refrigeration and freezing. It is well known that, for drugs with high safety requirements, storage and transportation conditions cannot be drastically changed, so complex storage and transportation conditions will generate huge costs, such as cold storage and professional cold chain transportation, which will be attached to the drug price and ultimately increase the burden of drug users. If the problem of drug stability can be overcome to use conventional and simple shelf and transportation conditions, it will greatly benefit the drug users. A storage condition of the tertiary amine pharmaceutical composition of the present invention is 5°C±3°C, or 20°C±5°C, or 25°C±5°C, and is preferably 20°C±5°C.

A storage condition of the N1 composition is 5°C±3°C, or 20°C±5°C. In some embodiments, the storage condition of the N1 composition is 5°C±3°C, and a storage time of the N1 composition is no less than 36 months. In some embodiments, the storage condition of the N1 composition is 20°C±5°C, and the storage time of the N1 composition is no less than 24 months.

The present invention provides the tertiary amine pharmaceutical composition and the industrialized batch preparation method thereof. The content of the impurities generated by the reaction between the drug with tertiary amine structure and the halogenated hydrocarbons meet ICH requirements, and the content of the impurities cannot increase or increase slowly during storage under conventional conditions, or do not exceed ICH requirements within the validity period of identification.

### BRIEF DESCRIPTION OF THE DRAWINGS

FIG. 1 is the mass spectrogram of an impurity generated when N1 reacts with dichloromethane.

### DETAILED DESCRIPTION

The present invention provides a tertiary amine pharmaceutical composition, comprising a drug with tertiary amine structure, a biocompatible polymer material, and quaternary ammonium salt impurities. The pharmaceutical composition is obtained by dissolving or dispersing the drug in a halogenated hydrocarbon or a mixed solvent mainly containing halogenated hydrocarbon or a solution containing halogenated hydrocarbon, and the quaternary ammonium salt impurities are generated from reaction of the drug with tertiary amine structure with the halogenated hydrocarbon. Through research, it is found by the present application that, by controlling the content of the quaternary ammonium salt impurities and the content of residual halogenated hydrocarbon in the tertiary amine pharmaceutical composition, the content of the impurities cannot increase or increase slowly during storage under conventional conditions, or do not exceed ICH requirements within the validity period of identification.

In the following embodiments, N1 is used as a model drug and dichloromethane is used as a halogenated hydrocarbon to describe the solutions of the present invention in detail. When N1 reacts with the dichloromethane, a structure of an impurity generated is:

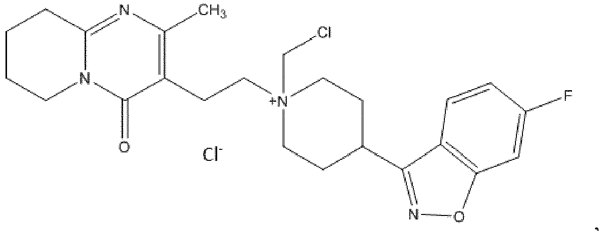

The mass spectrogram of the impurity is as shown in FIG. 1, and the RRT of the impurity by liquid phase detection is 1.5, wherein detection conditions of the impurity are:
(1) chromatographic column: Agilent ZORBAX SB-C18, 150×4.60 mm, 5 µm or equivalent chromatographic column;
(2) mobile phase: acetonitrile: trifluoroacetic acid: water = 20: 0.15: 80 (pH 3.0)
(3) flow rate: 1.5 mL/min
(4) column temperature: 35°C
(5) detection wavelength: 275 nm

Under these detection conditions, there may also be known impurities U1, U2 and U3 in the N1 pharmaceutical composition, with RRT of 0.7, 1.6 and 1.7 respectively.

### Embodiment 1 Preparation of N1-containing composition:

A formulation process for manufacturing 100,000 to 500,000 dose batches (a specific batch quantity of each batch was determined according to test parameters) of N1 composition shown in the following table, and the preparation method was:
(1) preparing 0.1wt% to 1.5wt% polyvinyl alcohol aqueous solution, and cooling to 5+/-3°C for later use;
(2) completely dissolving N1 (40wt%) and PLGA (60wt%) in about 4 times (V/w) of dichloromethane under mechanical stirring, and then filtering the mixture by a filter membrane with a pore diameter of 0.2 µm at a temperature not exceeding 20°C;
(3) after the step (2) was completed, slowly injecting the solution into the solution of step (1) for high-speed homogenization to form an O/W emulsion with a flow rate ratio of 1: 100, and an ambient temperature no more than 25°C;
(4) mechanically stirring the emulsion of step(3) to remove the dichloromethane and filtering to obtain a solidified spherical or non-spherical particle, filtering and changing water(room temperature, equal volume) twice in the middle of the process for equal time;
(5) washing the particles of step (4) with water for injection at 5 times the volume not exceeding 10°C to remove the polyvinyl alcohol on the surface of the particles;
(6) freeze-drying the particle of step (5) to obtain the composition, wherein the freeze-drying method was:

| | |
|---|---|
| pre-freezing | -30°C, 6 hours |
| primary drying | -20°C to -5°C, 8 hours, 200 mtorr; |
| secondary drying | 35°C, 24 hours, 100 mtorr |

(7) filling the particle of step (6) in a penicillin bottle sealed with a rubber stopper with a unit dose of 50 mg (calculated on the basis of N1), and then sealing the penicillin bottle with a moisture-proof aluminum foil bag for storage.

By adjusting the time used in step (2) and step (3) and controlling the duration of step (4), and adjusting and controlling the ambient temperature and the system temperature, drug particles with different residual amounts of halogenated hydrocarbon were obtained, and the influences of these indicators on the content control of RRT1.5 in the composition were investigated.

**Table 1 Formulations, processes and parameters of composition**

| Batch | PLGA parameters | Time spent in step (2) | Time spent in step (3) | Duration of step (4) | Ambient temperature and solution/suspension/emulsion temperature in step (4) |
|---|---|---|---|---|---|
| A1 | 100/0, 20 kDa, 0.20 dL/g | 8h | 3h | 48h | 20°C |
| A2 | 100/0, 25 kDa, 0.24 dL/g | 6h | 3h | 48h | 20°C |
| A3 | 100/0, 30 kDa, 0.29 dL/g | 4h | 3h | 48h | 20°C |
| A4 | 100/0, 35 kDa, 0.34 dL/g | 3h | 3h | 48h | 20°C |
| A5 | 75/25, 35 kDa, 0.34 dL/g | 3h | 6h | 48h | 20°C |
| A6 | 75/25, 40 kDa, 0.41 dL/g | 3h | 5h | 48h | 20°C |
| A7 | 75/25, 50 kDa, 0.52 dL/g | 3h | 4h | 48h | 20°C |
| A8 | 75/25, 60 kDa, 0.63 dL/g | 3h | 3h | 48h | 20°C |
| A9 | 95/5, 30 kDa, 0.29 dL/g | 3h | 3h | 24h | 20°C |
| A10 | 95/5, 35 kDa, 0.34 dL/g | 3h | 3h | 30h | 20°C |
| A11 | 95/5, 40 kDa, 0.41 dL/g | 3h | 3h | 36h | 20°C |
| A12 | 95/5, 45 kDa, 0.46 dL/g | 3h | 3h | 48h | 20°C |
| A13 | 85/15, 30 kDa, 0.31 dL/g | 3h | 3h | 48h | 35°C |
| A14 | 85/15, 35 kDa, 0.35 dL/g | 3h | 3h | 48h | 30°C |
| A15 | 85/15, 40 kDa, 0.40 dL/g | 3h | 3h | 48h | 25°C |
| A16 | 85/15, 45 kDa, 0.45 dL/g | 3h | 3h | 48h | 20°C |

### Embodiment 2

By studying results of different batches of products obtained in embodiment 1, the influences of the time spent in step (2) and the time spent in step (3) on the content of RRT1.5 were discussed. The results were shown in Table 2.

**Table 2 Changes of RRT1.5 impurity in different processes**

| Process parameters | | | Content of RRT1.5 impurity/% | Content of halogenated hydrocarbon/% | Particle size scope/µm |
|---|---|---|---|---|---|
| Time spent in step (2)/h | A1 | 8 | 0.08 | 0.95 | 20 to 89 |
| | A2 | 6 | 0.04 | 0.91 | 25 to 107 |
| | A3 | 4 | 0.01 | 0.90 | 30 to 98 |
| | A4 | 3 | - | 0.94 | 28 to 114 |
| Time spent in step (3)/h | A5 | 6 | 0.03 | 0.96 | 35 to 137 |
| | A6 | 5 | 0.02 | 0.90 | 22 to 96 |
| | A7 | 4 | - | 0.91 | 38 to 106 |
| | A8 | 3 | - | 0.93 | 31 to 110 |

| | | | | | |
|---|---|---|---|---|---|
| "-" means no impurity was detected or the content of the impurity was lower than a quantitative limit. | | | | | |

It can be seen from the data in Table 2 that when other conditions are constant, the content of RRT1.5 impurity obviously decreases with the decrease of the time spent in step (2) or step (3).

### Embodiment 3

Influences of the duration of step (4), the ambient environment and the system temperature on the content of RRT1.5 were further studied. The results were shown in Table 3.

**Table 3 Changes of RRT1.5 impurity in different processes**

| Process parameters | | | Content of RRT1.5 | Content of halogenated hydrocarbon | Particle size scope/µm |
|---|---|---|---|---|---|
| Duration of step (4)/h | A9 | 24 | - | 1.15 | 30 to 105 |
| | A10 | 30 | 0.00 | 0.91 | 32 to 94 |
| | A11 | 36 | - | 0.70 | 27 to 96 |
| | A12 | 48 | 0.01 | 0.64 | 26 to 124 |
| Ambient environment and system temperature of step (4)/°C | A13 | 35 | 0.13 | 068 | 20 to 85 |
| | A14 | 30 | 0.07 | 0.70 | 33 to 114 |
| | A15 | 25 | 0.02 | 0. 67 | 39 to 107 |
| | A16 | 20 | - | 0.63 | 34 to 122 |

| | | | | | |
|---|---|---|---|---|---|
| "-" means no impurity was detected or the content of the impurity was lower than a quantitative limit. | | | | | |

It can be seen from the data in Table 3 that when steps (2) and (3) are preferred conditions, the content of the RRT1.5 impurity does not change significantly with the increase of the duration of step (4), but organic solvent residues decrease obviously with the increase of time; when the durations of step (2), step (3) and step (4) are preferred conditions, the content of the RRT1.5 impurity obviously decreases with the decrease of the ambient environment and the system temperature of step (4).

### Embodiment 4

### Preparation of N1-containing composition:

A formulation process for manufacturing 100,000 to 500,000 dose batches (a specific batch quantity of each batch was determined according to test parameters) of N1 composition was shown in Table 4, wherein the preparation method was the same as that of Embodiment 1. Meanwhile, N1 compositions with different contents of RRT1.5 impurity and different contents of halogenated hydrocarbon were obtained by adjusting the process parameters.

**Table 4 Formulation and parameters of composition**

| Batch | PLGA parameters | Rate of charge of PLGA | Rate of charge of N1 | Content of RRT1.5 impurity | Content of halogenated hydrocarbon | Particle size scope/µm |
|---|---|---|---|---|---|---|
| B1 | 100/0, 20 kDa, 0.2 dL/g | 40% | 60% | 0.10% | 0.98% | 23 to 115 |
| B2 | 95/5, 25 kDa, 0.24 dL/g | 80% | 20% | 0.07% | 1.03% | 32 to 110 |
| B3 | 85/15, 30 kDa, 0.29 dL/g | 60% | 40% | 0.05% | 1.00% | 20 to 95 |
| B4 | 85/15, 35 kDa, 0.34 dL/g | 65% | 35% | 0.04% | 1.07% | 35 to 123 |
| B5 | 85/15, 40 kDa, 0.40 dL/g | 60% | 40% | 0.03% | 1.15% | 40 to 137 |
| B6 | 75/25, 50 kDa, 0.52 dL/g | 65% | 35% | 0.02% | 1.05% | 28 to 106 |
| B7 | 70/30, 55 kDa, 0.57 dL/g | 50% | 50% | 0.01% | 0.92% | 22 to 89 |
| B8 | 85/15, 35 kDa, 0.34 dL/g | 65% | 35% | 0.04% | 2.01% | 30 to 104 |
| B9 | 85/15, 35 kDa, 0.34 dL/g | 65% | 35% | 0.04% | 1.53% | 37 to 131 |
| B10 | 85/15, 35 kDa, 0.34 dL/g | 65% | 35% | 0.04% | 1.25% | 30 to 118 |
| B11 | 85/15, 35 kDa, 0.34 dL/g | 65% | 35% | 0.04% | 0.71% | 26 to 104 |
| B12 | 85/15, 35 kDa, 0.34 dL/g | 65% | 35% | 0.04% | 0.60% | 31 to 98 |

### Embodiment 5

Under a storage condition of 5°C±3°C, a residual amount of a halogenated hydrocarbon was about 1%, and influences of the residual amount of the halogenated hydrocarbon on a content change of an RRT1.5 impurity during storage were shown in Table 5.

**Table 5 Changes of RRT1.5 impurity during storage at 5°C±3°C**

| Storage time/month | B1 | B2 | B3 | B4 | B5 | B6 | B7 |
|---|---|---|---|---|---|---|---|
| 0 | 0.10% | 0.07% | 0.05% | 0.04% | 0.03% | 0.02% | 0.01% |
| 1 | 0.11% | 0.09% | 0.06% | 0.04% | 0.03% | 0.02% | 0.01% |
| 3 | 0.15% | 0.12% | 0.08% | 0.06% | 0.04% | 0.02% | 0.01% |
| 6 | 0.19% | 0.15% | 0.10% | 0.08% | 0.06% | 0.03% | 0.02% |
| 12 | 0.22% | 0.19% | 0.13% | 0.11% | 0.07% | 0.04% | 0.02% |
| 18 | 0.29% | 0.24% | 0.15% | 0.13% | 0.09% | 0.06% | 0.02% |
| 24 | 0.35% | 0.29% | 0.19% | 0.16% | 0.13% | 0.08% | 0.02% |

According to the changes of the RRT1.5 impurity in Table 5, when the initial content of the RRT1.5 impurity is higher than 0.05%, the contents increase to more than 0.2% after 18 months of storage, and when the initial content of the RRT1.5 impurity is less than 0.05%, all the contents are lower than 0.2% after 24 months of storage, and the smaller the initial content of the RRT1.5 impurity is, the slower the increase is. When the initial content of the RRT1.5 impurity is less than 0.02%, there is no obvious increase within 24 months.

### Embodiment 6

Under a storage condition of 20°C±5°C, a residual amount of a halogenated hydrocarbon was about 1%, and influences of the residual amount of the halogenated hydrocarbon on a content change of the RRT1.5 impurity during storage were shown in Table 6.

**Table 6 Changes of RRT1.5 impurity during storage at 20°C±5°C**

| Storage time/month | B1 | B2 | B3 | B4 | B5 | B6 | B7 |
|---|---|---|---|---|---|---|---|
| 0 | 0.10% | 0.07% | 0.05% | 0.04% | 0.03% | 0.02% | 0.01% |
| 1 | 0.15% | 0.11% | 0.06% | 0.05% | 0.04% | 0.02% | 0.01% |
| 3 | 0.19% | 0.16% | 0.08% | 0.07% | 0.05% | 0.03% | 0.02% |
| 6 | 0.25% | 0.21% | 0.10% | 0.09% | 0.08% | 0.05% | 0.03% |
| 12 | 0.30% | 0.24% | 0.13% | 0.11% | 0.11% | 0.07% | 0.05% |
| 18 | 0.36% | 0.29% | 0.16% | 0.14% | 0.13% | 0.08% | 0.06% |
| 24 | 0.42% | 0.34% | 0.20% | 0.18% | 0.16% | 0.11% | 0.08% |

According to the changes of the RRT1.5 impurity in Table 6, compared with the storage condition of 5°C ±3°C, when the initial content of the RRT1.5 impurity is higher than 0.05%, the content increases more obviously during the storage at 20°C±5°C, and the content exceeds 0.2% after 6 months; when the initial content of the RT1.5 impurity is less than 0.03%, the content is still less than 0.2% within 24 months, and when the initial content of the RRT1.5 impurity is less than 0.02%, the increase rate is relatively slow within 24 months.

### Embodiment 7

Under a storage condition of 20°C±5°C, an initial content of the RRT1.5 impurity was 0.04%, and influences of a residual amount of a halogenated hydrocarbon on a content change of the impurity were shown in Table 7.

**Table 7 Changes of RRT1.5 impurity during storage at 20°C±5°C**

| Storage time/month | B4 | B8 | B9 | B10 | B11 | B12 |
|---|---|---|---|---|---|---|
| 0 | 0.04% | 0.04% | 0.04% | 0.04% | 0.04% | 0.04% |
| 1 | 0.05% | 0.08% | 0.07% | 0.05% | 0.05% | 0.04% |
| 3 | 0.07% | 0.15% | 0.13% | 0.07% | 0.06% | 0.05% |
| 6 | 0.09% | 0.23% | 0.20% | 0.11% | 0.08% | 0.05% |
| 12 | 0.11% | 0.27% | 0.25% | 0.14% | 0.10% | 0.06% |
| 18 | 0.14% | 0.33% | 0.29% | 0.17% | 0.13% | 0.07% |
| 24 | 0.18% | 0.38% | 0.34% | 0.20% | 0.15% | 0.08% |

According to the changes of the RRT1.5 impurity in Table 7, when the content of the halogenated hydrocarbon is higher than 1.5%, the RRT1.5 impurity increases more obviously during the storage at 20°C ±5°C, and the content exceeds 0.2% after 9 months; when the initial content of the RT1.5 impurity is less than 1.1%, the content is still less than 0.2% within 24 months, and when the initial content of the RRT1.5 impurity is less than 0.70%, the increase rate is not obvious within 24 months.

### Embodiment 8

Under a storage conditions of 5°C±3°C and 20°C±5°C, influences of a content of a halogenated hydrocarbon on U1, U2 and U3 impurities were shown in Table 8.

**Table 8 Changes of U1, U2 and U3 impurities during storage**

| Impurity | Batch of composition | Content of impurity/% | | | | | | |
|---|---|---|---|---|---|---|---|---|
| | | 0 month | 5°C±3°C | | | 20°C±5°C | | |
| | | | 12 months | 24 months | 36 months | 12 months | 24 months | 36 months |
| U1 | B1 | - | - | - | - | - | - | - |
| | B8 | - | - | - | - | - | - | - |
| | B9 | 0.02 | 0.01 | 0.01 | 0.02 | 0.01 | 0.01 | 0.01 |
| | B10 | - | - | - | - | - | - | - |
| | B11 | - | - | - | - | - | - | - |
| | B12 | 0.03 | 0.03 | 0.03 | 0.03 | 0.04 | 0.03 | 0.03 |
| U2 | B1 | 0.01 | - | 0.01 | 0.01 | 0.01 | 0.01 | 0.01 |
| | B8 | 0.02 | 0.01 | 0.02 | 0.02 | 0.01 | 0.01 | 0.02 |
| | B9 | - | - | - | - | - | - | - |
| | B10 | - | - | - | - | - | - | - |
| | B11 | - | - | - | - | - | - | - |
| | B12 | - | - | - | - | - | - | - |
| U3 | B1 | - | - | - | - | - | - | - |
| | B8 | - | - | - | - | - | - | - |
| | B9 | 0.01 | 0.01 | 0.01 | 0.01 | - | 0.01 | 0.02 |
| | B10 | - | - | - | - | - | - | - |
| | B11 | - | - | - | - | - | - | - |
| | B12 | - | - | - | - | - | - | - |

| | | | | | | | | |
|---|---|---|---|---|---|---|---|---|
| "-" means no impurity was detected or the content of the impurity was lower than a quantitative limit. | | | | | | | | |

It can be seen from the data in Table 8 that in the N1 composition, three impurities, U1, U2 and U3, have almost no change during storage, and have no visible relationship with the content of the halogenated hydrocarbon in the composition.

### Embodiment 9

According to the preparation method of Embodiment 1, sustained-release microspheres of entecavir, oxybutynin, raloxifene, rivastigmine, naloxone, naltrexone, buprenorphine, selegiline, buspirone, blonanserin, asenapine, olanzapine, lurasidone, N2 and N3 were prepared. The compositions were shown in Table 9. The particle size scope of the microspheres was controlled to be 30 µm to 120 µm, the content of a quaternary ammonium salt impurities was less than 0.05%, and the content of a halogenated hydrocarbon was about 0.69 to 0.9%. Changes of the quaternary ammonium salt impurities stored at 5°C±3°C and 20°C ±5°C for 12 months, 24 months and 36 months were investigated. The results were shown in Table 9.

**Table 9 Parameters of pharmaceutical composition containing drug with tertiary amine structure**

| Batch | Drug containing a tertiary amine structure | Content of drug | PLGA | Content of the quaternary ammonium salt impurities | Content of halogenated hydrocarbon |
|---|---|---|---|---|---|
| C1 | Entecavir | 28.5% | 85/15, 33 kDa, 0.32 dL/g | - | 0.70% |
| C2 | Oxybutynin | 30% | 95/5, 25 kDa, 0.24 dL/g | 0.02% | 0.69% |
| C3 | Raloxifene | 35% | 85/15, 30 kDa, 0.29 dL/g | - | 0.75% |
| C4 | Rivastigmine | 40% | 85/15, 40 kDa, 0.41 dL/g | - | 0.80% |
| C5 | Naloxone | 25% | 75/25, 45 kDa, 0.46 dL/g | 0.01% | 0.85% |
| C6 | Naltrexone | 42% | 75/25, 50 kDa, 0.52 dL/g | 0.02% | 0.84% |
| C7 | Buprenorphine | 36% | 70/30, 55 kDa, 0.57 dL/g | 0.03% | 0.86% |
| C8 | Selegiline | 30% | 85/15, 35 kDa, 0.34 dL/g | - | 0.85% |
| C9 | Buspirone | 20% | 85/15, 35 kDa, 0.34 dL/g | 0.01% | 0.78% |
| C10 | Blonanserin | 40% | 80/20, 35 kDa, 0.34 dL/g | 0.04% | 0.90% |
| C11 | Asenapine | 25% | 80/20, 35 kDa, 0.34 dL/g | - | 0.82% |
| C12 | Olanzapine | 55% | 85/15, 30 kDa, 0.30 dL/g | - | 0.90% |
| C13 | Lurasidone | 45% | 90/10, 25 kDa, 0.24 dL/g | - | 0.73% |
| C14 | N2 | 50% | 95/5, 25 kDa, 0.24 dL/g | 0.03% | 0.90% |
| C15 | N3 | 60% | 100/0, 20 kDa, 0.2 dL/g | 0.04% | 0.70% |

| | | | | | |
|---|---|---|---|---|---|
| "-" means no impurity was detected or the content of the impurity was lower than a quantitative limit. | | | | | |

**Table 10 Changes of the quaternary ammonium salt impurities in compositions of batches C1 to C15 during storage at 20°C±5°C**

| Batch of composition | Content of the quaternary ammonium salt impurities/% | | | | | | |
|---|---|---|---|---|---|---|---|
| | 0 month | 5°C±°C | | | 20°C±°C | | |
| | | 12 months | 24 months | 36 months | 12 months | 24 months | 36 months |
| C1 | - | - | 0.01 | 0.2 | 0.01 | 0.3 | 0.05 |
| C2 | 0.02 | 0.02 | 0.03 | 0.04 | 0.03 | 0.05 | 0.08 |
| C3 | - | - | 0.01 | 0.03 | 0.02 | 0.05 | 0.07 |
| C4 | - | - | 0.02 | 0.05 | 0.02 | 0.04 | 0.08 |
| C5 | 0.01 | 0.02 | 0.04 | 0.08 | 0.03 | 0.07 | 0.10 |
| C6 | 0.02 | 0.03 | 0.07 | 0.09 | 0.05 | 0.08 | 0.12 |
| C7 | 0.03 | 0.03 | 0.06 | 0.09 | 0.08 | 0.13 | 0.17 |
| C8 | - | - | 0.02 | 0.05 | 0.01 | 0.04 | 0.10 |
| C9 | 0.01 | 0.01 | 0.03 | 0.06 | 0.02 | 0.05 | 0.09 |
| C10 | 0.04 | 0.05 | 0.07 | 0.08 | 0.08 | 0.12 | 0.16 |
| C11 | - | - | 0.01 | 0.03 | 0.02 | 0.05 | 0.08 |
| C12 | - | - | 0.01 | 0.05 | 0.02 | 0.08 | 0.15 |
| C13 | - | - | - | 0.02 | - | 0.03 | 0.07 |
| C14 | 0.03 | 0.04 | 0.07 | 0.11 | 0.06 | 0.11 | 0.19 |
| C15 | 0.04 | 0.04 | 0.05 | 0.07 | 0.07 | 0.10 | 0.14 |

| | | | | | | | |
|---|---|---|---|---|---|---|---|
| "-" means no impurity was detected or the content of the impurity was lower than a quantitative limit. | | | | | | | |

It can be seen from the changes of the quaternary ammonium salt impurities of each composition in Table 9 that when the content of the halogenated hydrocarbon content does not exceed 0.9% and the initial value of the quaternary ammonium impurities does not exceed 0.04%, the quaternary ammonium impurities of the composition is still less than 0.2% during storage at two temperatures. Meanwhile, when the content of the halogenated hydrocarbon is larger or the initial value of the quaternary ammonium salt impurities is higher, the increase of the quaternary ammonium salt impurities is larger during storage at two temperatures.

## Claims

1. A tertiary amine pharmaceutical composition, comprising a drug with tertiary amine structure, a biocompatible polymer material, and a quaternary ammonium salt impurity, the pharmaceutical composition being obtained by dissolving or dispersing the drug in a halogenated hydrocarbon or a mixed solvent mainly containing halogenated hydrocarbon or a solution containing halogenated hydrocarbon, and the quaternary ammonium salt impurities being generated from a reaction of the drug with tertiary amine structure and the halogenated hydrocarbon, **characterized in that**, the pharmaceutical composition comprises 40wt% to 80wt% of the biocompatible polymer material, and 20% to 60wt% of the drug with tertiary amine structure; a content of the quaternary ammonium salt impurities is less than 0.05wt%; and a content of the halogenated hydrocarbon in the tertiary amine pharmaceutical composition is less than 1.5wt%.

2. The tertiary amine pharmaceutical composition as claimed in claim 1, **characterized in that**, the drug with tertiary amine structure comprises any one of entecavir, oxybutynin, raloxifene, rivastigmine, naloxone, naltrexone, buprenorphine, selegiline, buspirone, blonanserin, asenapine, olanzapine, lurasidone, 3-[2-[4-(6-fluoro-1,2-benzisoxazole-3-yl)-1-piperidinyl] ethyl]-6,7,8,9-tetrahydro-2-methyl-4H-pyridine[1,2-α]pyrimidine-4-keton, 6,7,8,9-tetrahydro-3-(2-(4-(6-fluoro-1,2-benzisoxazole-3-yl)-1-piperidinyl)ethyl)-9-hydroxy-2-methyl-4H-pyridine[2,1-a]pyrimidine-4-one and (±)-3-[2-[4-[6-fluoro-1,2-benzisoxazole-3-yl)-1-piperidine]ethyl]-6,7,8,9-tetrahydro-2-methyl-4-oxo-4H-py ridine[1,2-a]pyrimidine-9-yl palmitate.

3. The tertiary amine pharmaceutical composition as claimed in claim 1, **characterized in that**, the biocompatible polymer material is PLGA, a ratio of LA to GA of the PLGA is 100/0 to 50/50, a molecular weight of the PLGA is 20 kDa to 60 kDa, and an intrinsic viscosity of the PLGA is 0.2 dL/g to 0.65 dL/g.

4. The tertiary amine pharmaceutical composition as claimed in claim 1, **characterized in that**, the drug with tertiary amine structure is any one of 3-[2-[4-(6-fluoro-1,2-benzisoxazole-3-yl)-1-piperidinyl] ethyl]-6,7,8,9-tetrahydro-2-methyl-4H-pyridine[1,2-α]pyrimidine-4-keton, 6,7,8,9-tetrahydro-3-(2-(4-(6-fluoro-1,2-benzisoxazole-3-yl)-1-piperidinyl)ethyl)-9-hydroxy-2-methyl-4H-pyridine[2,1-a]pyrimidine-4-one and (±)-3-[2-[4-[6-fluoro-1,2-benzisoxazole-3-yl)-1-piperidine]ethyl]-6,7,8,9-tetrahydro-2-methyl-4-oxo-4H-py ridine[1,2-a]pyrimidine-9-yl palmitate, and the biocompatible polymer material is at least one of PLGA, chloroform or dichloromethane; and the content of the quaternary ammonium salt impurities is less than 0.04wt%.

5. The tertiary amine pharmaceutical composition as claimed in claim 3, **characterized in that**, the biocompatible polymer material is PLGA, the ratio of LA to GA of the PLGA is 100/0 to 70/30, the molecular weight of the PLGA is 20 kDa to 55 kDa, and the intrinsic viscosity of the PLGA is 0.2 dL/g to 0.57 dL/g.

6. The tertiary amine pharmaceutical composition as claimed in claim 1, **characterized in that**, the drug with tertiary amine structure is 3-[2-[4-(6-fluoro-1,2-benzisoxazole-3-yl)-1-piperidinyl] ethyl]-6,7,8,9-tetrahydro-2-methyl-4H-pyridine[1,2-a]pyrimidine-4-keton with a content of 30wt% to 50wt%; the biocompatible polymer material is PLGA with a content of 50wt% to 70wt%; and a structural formula of the impurity is as shown by Formula (1): 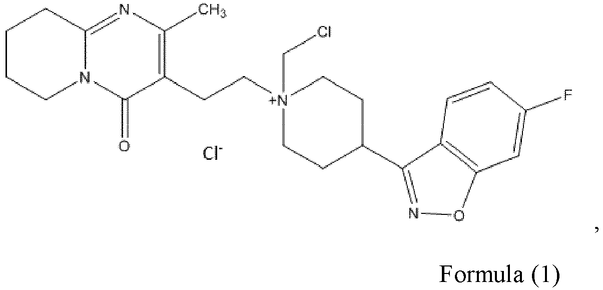 wherein, the content of the impurity is less than 0.03wt%.

7. The tertiary amine pharmaceutical composition as claimed in claim 6, **characterized in that**, a ratio of LA to GA of the PLGA is 95/05 to 70/30, a molecular weight of the PLGA is 25 kDa to 50 kDa, and an intrinsic viscosity of the PLGA is 0.24 dL/g to 0.52 dL/g.

8. The tertiary amine pharmaceutical composition as claimed in any one of claims 1 to 7, **characterized in that**, the tertiary amine pharmaceutical composition is a spherical or non-spherical particle with a major axis of 10 µm to 200 µm.

9. An application of the tertiary amine pharmaceutical composition as claimed in any one of claims 1 to 7 in preparing a drug for resisting anxiety and depression, and treating acute and chronic schizophrenia and other obvious positive and negative symptoms of various psychotic states, **characterized in that**, the drug with tertiary amine structure is any one of buspirone, blonanserin, asenapine, olanzapine, lurasidone, 3-[2-[4-(6-fluoro-1,2-benzisoxazole-3-yl)-1-piperidinyl] ethyl]-6,7,8,9-tetrahydro-2-methyl-4H-pyridine[1,2-α]pyrimidine-4-keton, 6,7,8,9-tetrahydro-3-(2-(4-(6-fluoro-1,2-benzisoxazole-3-yl)-1-piperidinyl)ethyl)-9-hydroxy-2-methyl-4H-pyridine[2,1-a]pyrimidine-4-one and (±)-3-[2-[4-[6-fluoro-1,2-benzisoxazole-3-yl)-1-piperidine]ethyl]-6,7,8,9-tetrahydro-2-methyl-4-oxo-4H-py ridine[1,2-a]pyrimidine-9-yl palmitate.

10. An industrialized batch preparation method of the tertiary amine pharmaceutical composition as claimed in any one of claims 1 to 7, **characterized by** using an emulsion-solvent evaporation method for preparation, comprising the following steps of:
(1) preparing an aqueous solution with a surfactant, and cooling the aqueous solution for later use;
(2) dissolving or dispersing the drug with tertiary amine structure and the biocompatible polymer material in the halogenated hydrocarbon to form a solution or suspension, wherein a time spent is no more than 4 hours, a temperature of the solution is no more than 20°C±5°C, and no heating step is carried out;
(3) injecting the solution or suspension obtained in step (2) into the aqueous solution with the surfactant for emulsification to form O/W or S/O/W emulsion, wherein a time spent is no more than 4 hours, an ambient temperature is no more than 25°C, and no heating or vacuum step is carried out;
(4) mechanically stirring to remove the halogenated hydrocarbon in a product of step (3) to obtain a solidified spherical or non-spherical particles, wherein a duration for the mechanical stirring is no less than 24 hours, an ambient temperature and a system temperature are no more than 25°C, no heating or vacuum step is carried out, and a residual amount of the halogenated hydrocarbon in the obtained particles is no more than 2.0wt%;
(5) washing the particle of step (4) to remove the surfactant on the surface of the particles; and
(6) freeze-drying the particle of step (5) to obtain the tertiary amine pharmaceutical composition.

11. The method as claimed in claim 10, **characterized in that**, step (2) and step (3) are continuous processes, or a time between the two steps is no more than 1 hour.

12. The method as claimed in claim 10, **characterized in that**, in step (6), freeze-drying conditions are:
pre-freezing at -30°C to -45°C for 4 hours to 6 hours; primary drying at -20°C to -5°C for 8 hours to 16 hours under 100 mtorr to 200 mtorr; secondary drying at 30°C to 35°C for 18 hours to 30 hours under 100 mtorr to 200 mtorr; and the residual amount of the halogenated hydrocarbon in the obtained particle being no more than 1.5wt%

13. The method as claimed in claim 10, **characterized in that**, the industrialized batch refers to a batch with a single batch production cycle of 1 month /2 months, and no less than 500,000 doses.
